# EUROPÄISCHE PATENTSCHRIFT

(11) **EP 2 790 742 B1**
(45) Veröffentlichungstag und Bekanntmachung des Hinweises auf die Patenterteilung: **01.05.2019**
(21) Anmeldenummer: 14705041.3
(22) Anmeldetag: 14.02.2014
(51) Int. Cl.: A61L 9/22, F24F 3/16, H01T 23/00

(54) **RAUMLUFTREINIGUNGSGERÄT MIT PLASMAGENERATOR UND RADIALER AUSSTRÖMUNG**
INDOOR AIR PURIFICATION APPARATUS WITH PLASMA GENERATOR AND RADIAL EXHAUST
APPAREIL DE PURIFICATION DE L'AIR AMBIANT AVEX GENERATEUR DU PLASMA ET ECOULEMENT RADIALE

(30) Priorität: 27.02.2013 DE 202013001963 U
(43) Veröffentlichungstag der Anmeldung: 22.10.2014
(73) Patentinhaber: AL-KO Therm GmbH, 89343 Jettingen-Scheppach (DE)
(72) Erfinder: CZEJKA, Stephan, 89343 Jettingen-Scheppach (DE); DONG, Binjie, 89312 Günzburg (DE); GINSTERBLUM, Stefan, 89358 Kammeltal (DE)
(74) Vertreter: v. Bezold & Partner Patentanwälte - PartG mbB
(86) Internationale Anmeldenummer: PCT/EP2014/000416
(87) Internationale Veröffentlichungsnummer: WO 2014/102398

(56) Entgegenhaltungen:
- EP-A2- 2 719 960
- WO-A1-2010/109160
- WO-A1-2014/049128
- WO-A2-2013/174467
- DE-A1-102006 023 069
- DE-U1-202004 012 352
- GB-A- 2 273 048
- JP-A- 2009 285 654
- JP-A- 2010 017 520

## Beschreibung

Die Erfindung betrifft ein Raumluftreinigungsgerät zur Reinigung von Raumluft, insbesondere in einem Aufenthaltsraum, wie beispielsweise einem Wohnraum.

Aus dem Stand der Technik sind Raumluftreinigungsgeräte bekannt, bei denen die zu reinigende Raumluft mit einem nicht-thermischen atmosphärischen Plasma behandelt wird. Diese bekannten Raumluftreinigungsgeräte arbeiten jedoch noch nicht in vollständig befriedigender Weise.

Ferner ist zum Stand der Technik hinzuweisen auf GB 2 273 048 A, DE 20 2004 012 352 U1, DE 10 2006 023 069 A1, JP 2009 285654 A, JP 2010 017520 A und WO 2010/109160 A1. Diese Druckschriften offenbaren teilweise Raumluftreinigungsgeräte mit einem Rohrelement, wobei die zu reinigende Raumluft axial in das Rohrelement eintritt. In der Regel muss die zu reinigende Raumluft hierbei jedoch wieder axial aus dem Rohrelement austreten, was den Gestaltungsspielraum einengt.

Ferner offenbaren auch die nachveröffentlichten Patentanmeldungen EP 2 719 960 A2 und WO 2014/049128 A1 Luftreinigungsgeräte. Allerdings ist der Plasmagenerator hierbei nicht in der Wandung eines Rohrelements angeordnet.

Der Erfindung liegt deshalb die Aufgabe zugrunde, die bekannten Raumluftreinigungsgeräte mit einem Plasmagenerator entsprechend zu verbessern.

Diese Aufgabe wird durch ein erfindungsgemäßes Raumluftreinigungsgerät gemäß dem Hauptanspruch gelöst.

Die Erfindung umfasst die allgemeine technische Lehre, ein Gebläse in das Raumluftreinigungsgerät zu integrieren, wobei das Gebläse die zu reinigende Raumluft durch das Raumluftreinigungsgerät bläst.

In einer Variante der Erfindung bläst das Gebläse die zu reinigende Raumluft in das Raumluftreinigungsgerät hinein. In einer anderen Variante der Erfindung saugt das Gebläse die zu reinigende Raumluft dagegen in das Raumluftreinigungsgerät hinein. In einem bevorzugten Ausführungsbeispiel der Erfindung dient das Gebläse jedoch dazu, die zu reinigende Raumluft in den Bereich des Raumluftreinigungsgeräts mit dem Plasmagenerator hinein zu blasen.

In dem bevorzugten Ausführungsbeispiel der Erfindung ist der Plasmagenerator in der Weise ausgebildet, die aus der Patentanmeldung DE 10 2012 010 342 bekannt ist.

Das erfindungsgemäße Raumluftreinigungsgerät weist deshalb ein Rohrelement auf, wobei die zu reinigende Raumluft im Wesentlichen axial in das Rohrelement eintritt und im Wesentlichen radial durch die luftdurchlässige Wandung des Rohrelements wieder aus dem Rohrelement austritt.

Der Plasmagenerator ist hierbei in der Wandung des Rohrelements angeordnet, wie es aus der vorstehend genannten Patentanmeldung bekannt ist.

Darüber hinaus ist in der Wandung des Rohrelements vorzugsweise auch ein Adsorber angeordnet zur Adsorption von Verunreinigungen aus der Luft und zur Bildung einer Reaktionsoberfläche.

Ferner befindet sich der Wandung des Rohrelements vorzugsweise auch ein Katalysator zur katalytischen Umwandlung von Verunreinigungen in der Luft.

Diese Konstruktion eines Rohrelements mit einem Plasmagenerator, einem Adsorber und einem Katalysator in der Wandung des Rohrelements ist aus der vorstehend genannten Patentanmeldung DE 10 2012 010 342 bekannt, so dass hinsichtlich der Einzelheiten dieses Aufbaus auf diese Patentanmeldung verwiesen werden kann.

In dem bevorzugten Ausführungsbeispiel der Erfindung ist das Rohrelement im Wesentlichen senkrecht ausgerichtet, so dass die zu reinigende Raumluft im Wesentlichen senkrecht in das Rohrelement eintritt. Vorzugsweise tritt die zu reinigende Raumluft hierbei senkrecht von unten nach oben in das Rohrelement ein, es ist jedoch alternativ auch möglich, dass die zu reinigende Raumluft senkrecht von oben nach unten in das Rohrelement eintritt.

Weiterhin ist zu erwähnen, dass die Wandung des Rohrelements in dem bevorzugten Ausführungsbeispiel zylindrisch ist. Es ist jedoch alternativ auch möglich, dass das Rohrelement anders geformt ist, beispielsweise mit einem rechteckigen oder elliptischen Querschnitt.

In dem bevorzugten Ausführungsbeispiel weist das erfindungsgemäße Raumluftreinigungsgerät ferner einen schlauchförmigen Partikelfilter auf, der das Rohrelement außen umgibt und die aus der Wandung des Rohrelements austretende gereinigte Raumluft filtert.

Vorzugsweise ist dieser schlauchförmige Partikelfilter elastisch und auf das Rohrelement austauschbar aufgezogen. Dies bietet den Vorteil, dass der schlauchförmige Partikelfilter einfach ausgewechselt werden kann, indem der alte Partikelfilter abgezogen und ein neuer Partikelfilter aufgezogen wird.

Vorzugsweise handelt es sich bei dem Partikelfilter um einen sogenannten HEPA-Filter (HEPA: High Efficiency Particulate Airfilter), wobei derartige Filter an sich aus dem Stand der Technik bekannt sind und deshalb nicht näher beschrieben werden müssen. An dieser Stelle ist lediglich zu erwähnen, dass der Partikelfilter vorzugsweise mindestens 95% aller Partikel mit einer Partikelgröße von mehr als 0,3 µm aus der Raumluft ausfiltert.

Darüber hinaus weist das erfindungsgemäße Raumluftreinigungsgerät vorzugsweise einen Vorfilter auf, durch den die Raumluft vor dem Eintritt in den Plasmagenerator geleitet wird, um ein Zusetzen des Plasmagenerators zu vermeiden. Der Vorfilter ist also in dem erfindungsgemäßen Raumluftreinigungsgerät stromaufwärts vor dem Plasmagenerator angeordnet.

Darüber hinaus ist zu erwähnen, dass das Gebläse vorzugsweise in Strömungsrichtung zwischen dem Vorfilter und dem Rohrelement angeordnet ist, so dass das Gebläse die zu reinigende Raumluft durch den Vorfilter hindurch aus der Umgebung ansaugt und dann in das Rohrelement mit dem Plasmagenerator hineinbläst.

Hinsichtlich der äußeren Form des erfindungsgemäßen Raumluftreinigungsgeräts ist zu erwähnen, dass dieses vorzugsweise eine Stelenform aufweist. Bei dem erfindungsgemäßen Raumluftreinigungsgerät ist die Höhe also vorzugsweise wesentlich größer als die seitliche Erstreckung.

Darüber hinaus kann das erfindungsgemäße Raumluftreinigungsgerät eine Beleuchtungsanlage aufweisen, um Beleuchtungseffekte zu erzeugen, wozu vorzugsweise Leuchtdioden eingesetzt werden.

Die Beleuchtungsanlage wird vorzugsweise von einer Steuereinheit angesteuert, die mittels der Beleuchtungsanlage mehrfarbige Lichtsequenzen erzeugt.

Darüber hinaus enthält das erfindungsgemäße Raumluftreinigungsgerät vorzugsweise ein erstes Netzteil zur Stromversorgung von elektrischen Komponenten des Raumluftreinigungsgeräts aus einem Stromnetz, wobei es sich in der Regel um das übliche 230 V-Haushaltsnetz handelt. In dem bevorzugten Ausführungsbeispiel der Erfindung dient das erste Netzteil zur Stromversorgung des Gebläses. Zur Stromversorgung des Plasmagenerators ist dagegen vorzugsweise ein separates zweites Netzteil vorgesehen.

Ferner umfasst das erfindungsgemäße Raumluftreinigungsgerät vorzugsweise einen Ständer, um das Raumluftreinigungsgerät auf einem Untergrund aufstellen zu können, wobei der Ständer vorzugsweise eine Bodenplatte aufweist.

In dem bevorzugten Ausführungsbeispiel der Erfindung weist das Raumluftreinigungsgerät eine äußere Hülle auf, welche das Rohrelement außen umgibt und sich zumindest über die gesamte Länge des Rohrelements erstreckt, um das Rohrelement zu verhüllen, wobei ein Luftspalt zwischen dem Rohrelement und der Hülle frei bleibt, damit die aus dem Rohrelement radial austretende Luft in dem Luftspalt nach oben entweichen kann.

Hinsichtlich der Formgestaltung der Hülle bestehen vielfältige Möglichkeiten. So kann die Hülle beispielsweise zylindrisch, konkav oder konvex geformt sein.

In dem bevorzugten Ausführungsbeispiel kann die Hülle luftundurchlässig sein, wobei die Hülle unten eine Lufteintrittsöffnung und oben eine Luftaustrittsöffnung aufweist.

Schließlich weit das erfindungsgemäße Raumluftreinigungsgerät vorzugsweise einen Baugruppenträger ("Rack") auf, der das erste Netzteil, das zweite Netzteil, das Gebläse, den Vorfilter und/oder die Steuereinheit enthält. Dieser Baugruppenträger ist vorzugsweise mindestens teilweise innerhalb der Hülle angeordnet, so dass der Baugruppenträger das optische Erscheinungsbild des Raumluftreinigungsgeräts nicht stört.

Andere vorteilhafte Weiterbildungen der Erfindung sind in den Unteransprüchen gekennzeichnet oder werden nachstehend zusammen mit der Beschreibung der bevorzugten Ausführungsbeispiele der Erfindung anhand der Figuren näher erläutert. Es zeigen:
- Figur 1: eine Perspektivansicht eines erfindungsgemäßen Raumluftreinigungsgerätes mit einer demontierten äußeren Hülle,
- Figur 2: eine Perspektivansicht des Raumluftreinigungsgeräts gemäß Figur 1 mit einem zusätzlichen Ständer und einer äußeren Hülle,
- Figur 3: eine Perspektivansicht einer Abwandlung von Figur 2 mit einer konvexen Hülle, sowie
- Figur 4: eine Abwandlung von Figur 2 mit einer konkaven Hülle.

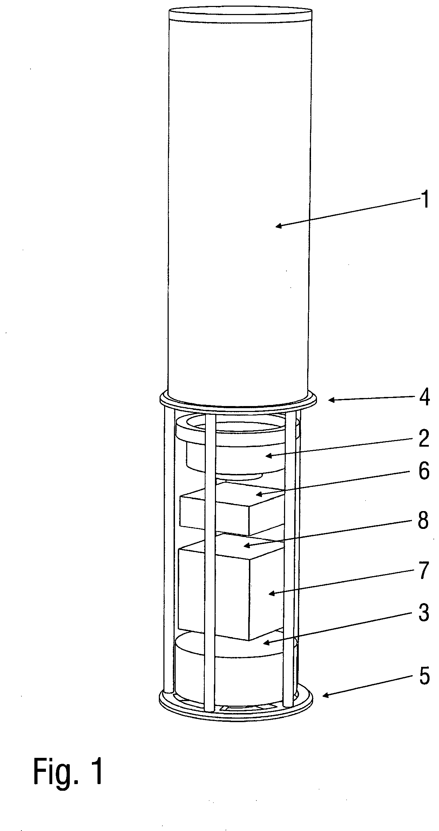

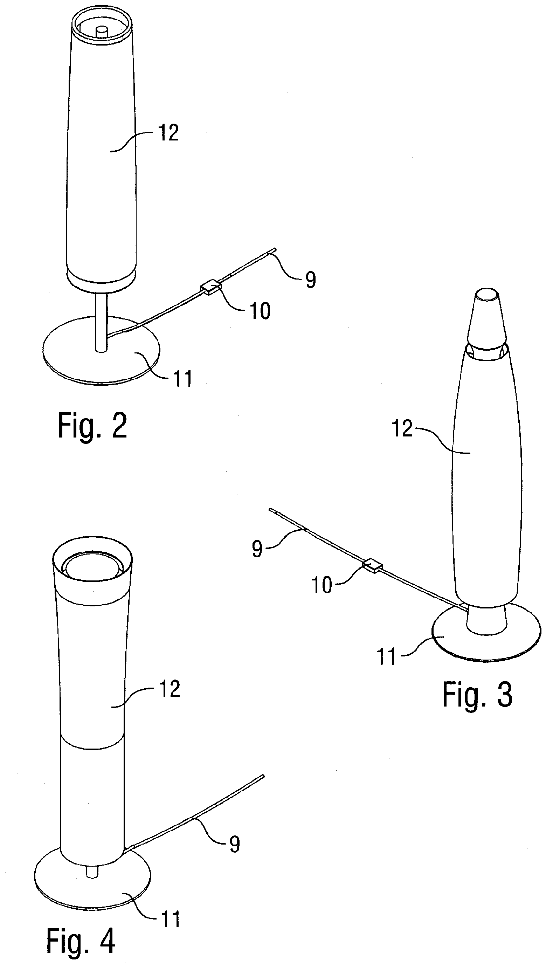

Die Figuren 1 und 2 zeigen ein bevorzugtes Ausführungsbeispiel eines erfindungsgemäßen Raumluftreinigungsgeräts, das beispielsweise in Wohnräumen zur Reinigung der Raumluft eingesetzt werden kann.

Das Raumluftreinigungsgerät weist als zentrales Reinigungselement ein Rohrelement 1 auf, wobei die zu reinigende Raumluft von einem Gebläse 2 axial von unten nach oben in das Rohrelement 1 hineingeblasen wird und das Rohrelement 1 dann wieder radial durch die luftdurchlässige Wandung des Rohrelements 1 hindurch verlässt.

In der Wandung des Rohrelements 1 ist ein Plasmagenerator angeordnet, der ein nicht-thermisches atmosphärisches Plasma zur Behandlung der zu reinigenden Raumluft erzeugt.

Darüber hinaus ist in der Wandung des Rohrelements 1 auch ein Adsorber angeordnet, um Verunreinigungen aus der Luft zu adsorbieren und eine Reaktionsoberfläche zu bilden.

Ferner befindet sich in der Wandung des Rohrelements 1 noch ein Katalysator zur katalytischen Umwandlung von Verunreinigungen in der Luft.

Es besteht jedoch im Rahmen der Erfindung auch die Möglichkeit, dass in der Wandung des Rohrelements 1 zusätzlich zu dem Plasmagenerator nur ein Adsorber oder nur ein Katalysator angeordnet ist.

Hinsichtlich des Aufbaus und der Funktionsweise des Rohrelements 1 mit dem Plasmagenerator, dem Adsorber und dem Katalysator wird auf die Patentanmeldung DE 10 2012 010 342 verwiesen.

Das Gebläse 2 saugt die zu reinigende Raumluft durch einen Vorfilter 3 hindurch aus der Umgebung an, wobei der Vorfilter 3 verhindern soll, dass der Plasmagenerator in der Wandung des Rohrelements 1 zusetzt.

Das Gebläse 2 und der Vorfilter 3 sind in einem Baugruppenträger 4 angeordnet, der eine Bodenplatte 5 aufweist.

Darüber hinaus beherbergt der Baugruppenträger 4 ein Netzteil 6 für das Gebläse 2, ein Netzteil 7 für den Plasmagenerator und eine Platine 8 zur Ansteuerung von Leuchtdioden einer Beleuchtungsanlage, wobei die Beleuchtungsanlage im Betrieb mehrfarbige Lichtsequenzen erzeugen kann, was jedoch nur eine ästhetische Bedeutung hat.

Auf das Rohrelement 1 wird im Betrieb ein elastischer schlauchförmiger HEPA-Filter (HEPA: High Efficiency Particulate Airfilter) aufgezogen, um feine Partikel aus der Luft auszufiltern, die durch die Wandung des Rohrelements 1 in radialer Richtung austritt. Der schlauchförmige HEPA-Filter kann in einfacher Weise ausgewechselt werden, indem der alte Filter von dem Rohrelement 1 abgezogen und ein neuer Filter auf das Rohrelement 1 aufgezogen wird.

Figur 2 zeigt zusätzlich, dass das Raumluftreinigungsgerät über ein Elektrokabel 9 und einen Schalter 10 an ein herkömmliches Stromnetz angeschlossen werden kann.

Darüber hinaus zeigt Figur 1 eine Bodenplatte 11 zum Aufstellen des Raumluftreinigungsgerätes auf einem Boden.

Schließlich ist aus Figur 2 noch ersichtlich, dass das Raumluftreinigungsgerät außen eine Hülle 12 aufweist, die in diesem Ausführungsbeispiel im Wesentlichen zylindrisch ausgebildet ist.

Die zu reinigende Raumluft tritt hierbei unten in die Hülle 12 ein und wird dann von dem Gebläse 2 durch den Vorfilter 3 angesaugt und in das Rohrelement 1 hineingeblasen.

Anschließend verlässt die gereinigte Raumluft die Hülle 12 wieder durch eine Luftauftrittsöffnung an der Oberseite der Hülle 12.

Hierbei ist zu erwähnen, dass zwischen der Hülle 12 und der Wandung des Rohrelements 1 ein ausreichender Luftspalt verbleibt, damit die gereinigte Raumluft nach oben entweichen kann.

Figur 3 zeigt eine Abwandlung des Ausführungsbeispiels gemäß den Figuren 1 und 2, so dass wir Vermeidung von Wiederholungen auf die vorstehende Beschreibung verwiesen wird, wobei für entsprechende Einzelheiten die selben Bezugszeichen verwendet werden.

Eine Besonderheit dieses Ausführungsbeispiels besteht darin, dass die Hülle 12 nicht zylindrisch ist, sondern konvex.

Figur 4 zeigt eine weitere Abwandlung, so dass zur Vermeidung von Wiederholungen wieder auf die vorstehende Beschreibung verwiesen wird, wobei für entsprechende Einzelheiten die selben Bezugszeichen verwendet werden.

Eine Besonderheit dieses Ausführungsbeispiels besteht, dass die Hülle 12 konkav geformt ist.

Die Erfindung ist nicht auf die vorstehend beschriebenen, bevorzugten Ausführungsbeispiele beschränkt. Vielmehr ist eine Vielzahl von Varianten und Abwandlungen möglich, die ebenfalls von dem Erfindungsgedanken Gebrauch machen und deshalb in den Schutzbereich fallen. Insbesondere beansprucht die Erfindung auch Schutz für den Gegenstand und die Merkmale der Unteransprüche unabhängig von den in Bezug genommenen Ansprüchen.

## Patentansprüche

1. Raumluftreinigungsgerät zur Reinigung von Raumluft, mit
a) einem Plasmagenerator zur Erzeugung eines nicht-thermischen Plasmas zur Behandlung der zu reinigenden Raumluft mit dem nicht-thermischen Plasma,
b) einem Gebläse (2) zum Blasen der zu reinigenden Raumluft durch das Raumluftreinigungsgerät, und
c) einem Rohrelement (1), wobei die zu reinigende Raumluft axial in das Rohrelement (1) eintritt,
**dadurch gekennzeichnet,**
d) dass die zu reinigende Raumluft radial durch die luftdurchlässige Wandung des Rohrelements (1) wieder aus dem Rohrelement (1) austritt, und
e) dass der Plasmagenerator in der Wandung des Rohrelements (1) angeordnet ist.

2. Raumluftreinigungsgerät nach Anspruch 1,
**gekennzeichnet durch**
a) einen Adsorber zur Adsorption von Verunreinigungen aus der Luft und zur Bildung einer Reaktionsoberfläche, wobei der Adsorber in der Wandung des Rohrelements (1) angeordnet ist, und/oder
b) einen Katalysator zur katalytischen Umwandlung von Verunreinigungen in der Luft, wobei der Katalysator in der Wandung des Rohrelements (1) angeordnet ist.

3. Raumluftreinigungsgerät nach einem der vorhergehenden Ansprüche, **dadurch gekennzeichnet,**
a) **dass** das Raumluftreinigungsgerät einen Ständer (11) aufweist zum Aufstellen des Raumluftreinigungsgeräts auf einem Untergrund,
b) **dass** das Rohrelement (1) bezüglich des Untergrunds senkrecht ausgerichtet ist, so dass die zu reinigende Raumluft senkrecht in das Rohrelement (1) eintritt, und
c) **dass** die zu reinigende Raumluft senkrecht von unten in das Rohrelement (1) eintritt, und
d) **dass** die Wandung des Rohrelements (1) zylindrisch ist.

4. Raumluftreinigungsgerät nach einem der vorhergehenden Ansprüche, **gekennzeichnet durch** einen schlauchförmigen Partikelfilter, der das Rohrelement (1) außen umgibt und die aus der Wandung des Rohrelements (1) austretende Raumluft filtert.

5. Raumluftreinigungsgerät nach Anspruch 4,
**dadurch gekennzeichnet,**
a) **dass** der schlauchförmige Partikelfilter elastisch ist und auf das Rohrelement (1) austauschbar aufgezogen ist, und/oder
b) **dass** der Partikelfilter ein HEPA-Filter ist, und/oder
c) **dass** der Partikelfilter aus der Raumluft mindestens 95% aller Partikel mit einer Partikelgröße von mehr als 0,3µm ausfiltert.

6. Raumluftreinigungsgerät nach einem der vorhergehenden Ansprüche, **gekennzeichnet durch** ein Vorfilter (3), durch den die Raumluft vor dem Eintritt in den Plasmagenerator geleitet wird, um ein Zusetzen des Plasmagenerators zu vermeiden.

7. Raumluftreinigungsgerät nach Anspruch 6, **dadurch gekennzeichnet, dass** das Gebläse (2) in Strömungsrichtung zwischen dem Vorfilter (3) und dem Rohrelement (1) angeordnet ist, so dass das Gebläse (2) die zu reinigende Raumluft durch den Vorfilter (3) hindurch aus der Umgebung ansaugt und in das Rohrelement (1) hinein bläst.

8. Raumluftreinigungsgerät nach einem der vorhergehenden Ansprüche, **gekennzeichnet durch**
a) eine Stelenform, und/oder
b) eine Beleuchtungsanlage zur Erzeugung von Beleuchtungseffekten, und/oder
c) eine Steuereinheit (8), welche die Beleuchtungsanlage zur Erzeugung mehrfarbiger Lichtsequenzen ansteuert.

9. Raumluftreinigungsgerät nach einem der vorhergehenden Ansprüche, **gekennzeichnet durch**
a) ein erstes Netzteil (6) zur Stromversorgung von elektrischen Komponenten des Raumluftreinigungsgeräts aus einem Stromnetz, und/oder
b) ein zweites Netzteil (7) zur Stromversorgung des Plasmagenerators.

10. Raumluftreinigungsgerät nach einem der vorhergehenden Ansprüche, **gekennzeichnet durch** eine Hülle (12), welche das Rohrelement (1) außen umgibt und sich zumindest über die gesamte Länge des Rohrelements (1) erstreckt, wobei ein Luftspalt zwischen dem Rohrelement (1) und der Hülle (12) frei bleibt.

11. Raumluftreinigungsgerät nach Anspruch 10,
**dadurch gekennzeichnet,**
a) **dass** die Hülle (12) zylindrisch ist, oder
b) **dass** die Hülle (12) konkav geformt ist, oder
c) **dass** die Hülle (12) konvex geformt ist.

12. Raumluftreinigungsgerät nach einem der Ansprüche 10 bis 11, **dadurch gekennzeichnet, dass** die Hülle (12) luftundurchlässig ist, aber unten eine Lufteintrittsöffnung und oben eine Luftaustrittsöffnung aufweist.

13. Raumluftreinigungsgerät nach einem der vorhergehenden Ansprüche, **gekennzeichnet durch** einen Baugruppenträger (4), der das erste Netzteil, das zweite Netzteil, das Gebläse (2), den Vorfilter (3) und/oder die Steuereinheit enthält.

14. Raumluftreinigungsgerät nach Anspruch 13, **dadurch gekennzeichnet, dass** der Baugruppenträger (4) mindestens teilweise in der Hülle (12) angeordnet ist.

## Claims

1. Room air cleaning device for cleaning room air, with
a) a plasma generator for generating a non-thermal plasma for treating the room air to be purified with the non-thermal plasma,
b) a blower (2) for blowing the room air to be cleaned through the room air cleaning device, and
c) a tubular element (1), the room air to be cleaned entering axially into the tubular element (1),
**characterized in that**
d) the room air to be cleaned leaves the tubular element (1) radially through the air-permeable wall of the tubular element (1), and
e) the plasma generator is arranged in the wall of the tubular element (1).

2. Room air cleaning device according to claim 1, **characterized by**
a) an adsorber for adsorbing impurities from the air and for forming a reaction surface, wherein the adsorber is arranged in the wall of the tubular element (1), and/or
b) a catalyst for the catalytic conversion of impurities in the air, the catalyst being arranged in the wall of the tubular element (1).

3. Room air cleaning device according to one of the preceding claims, **characterized in that**
a) the room air cleaning device has a stand (11) for placing the room air cleaning appliance on a ground,
b) the tubular element (1) is aligned vertically with respect to the ground so that the room air to be cleaned enters the tubular element (1) vertically, and
c) the room air to be cleaned enters the tubular element (1) vertically from below, and
d) the wall of the tubular element (1) is cylindrical.

4. Room air cleaning device according to one of the preceding claims, **characterised by** a tubular particle filter which surrounds the outside of the tubular element (1) and filters the room air emerging from the wall of the tubular element (1).

5. Room air cleaning device according to Claim 4, **characterized in that**
a) the tubular particle filter is elastic and is replaceably mounted on the tubular element (1), and/or
b) the particulate filter is a HEPA filter; and/or
c) the particulate filter filters out at least 95% of all particles with a particle size greater than 0,3µm from the ambient air.

6. Room air cleaning device according to one of the preceding claims, **characterized by** a prefilter (3) through which the room air is passed before entering the plasma generator to prevent clogging of the plasma generator.

7. Room air cleaning device according to claim 6, **characterised in that** the blower (2) is arranged in the direction of flow between the prefilter (3) and the tubular element (1), so that the blower (2) sucks the room air to be cleaned through the prefilter (3) from the environment and blows it into the tubular element (1).

8. Room air cleaning device according to one of the preceding claims, **characterised by**
a) a shape of a stelae; and/or
b) a lighting installation designed to produce lighting effects; and/or
c) a control unit (8) which controls the lighting installation for generating multicolour light sequences.

9. Room air cleaning device according to one of the preceding claims, **characterised by**
a) a first power supply unit (6) for supplying power to electrical components of the room air cleaning device from a mains supply, and/or
b) a second power supply unit (7) to supply power to the plasma generator.

10. Room air cleaning device according to one of the preceding claims, **characterized by** a sheath (12) which surrounds the tubular element (1) externally and extends at least over the entire length of the tubular element (1), leaving an air gap between the tubular element (1) and the sheath (12) free.

11. Room air cleaning device according to claim 10, **characterized in that**
a) the sheath (12) is cylindrical, or
b) the sheath (12) is concave shaped, or
c) the sheath (12) is convexly shaped.

12. Room air cleaning device according to any of claims 10 to 11, **characterized in that** the sheath (12) is air impermeable but has an air inlet opening at the bottom and an air outlet opening at the top.

13. Room air cleaning device according to one of the preceding claims, **characterized by** a subrack (4) which contains the first power supply, the second power supply, the fan (2), the prefilter (3) and/or the control unit.

14. Room air cleaning device according to claim 13, **characterized in that** the subrack (4) is arranged at least partially in the sheath (12).

## Revendications

1. Appareil de purification de l'air ambiant servant à purifier l'air ambiant, avec
a) un générateur de plasma servant à produire un plasma non thermique servant à traiter l'air ambiant à purifier avec le plasma non thermique,
b) une soufflante (2) servant à souffler l'air ambiant à purifier à travers l'appareil de purification de l'air ambiant, et
c) un élément tubulaire (1), dans lequel l'air ambiant à purifier entre de manière axiale dans l'élément tubulaire (1),
**caractérisé en ce**
d) que l'air ambiant à purifier sort à nouveau de l'élément tubulaire (1) de manière radiale à travers la paroi laissant passer l'air de l'élément tubulaire (1), et
e) que le générateur de plasma est disposé dans la paroi de l'élément tubulaire (1).

2. Appareil de purification de l'air ambiant selon la revendication 1,
**caractérisé par**
a) un adsorbeur servant à l'adsorption d'impuretés provenant de l'air et servant à former une surface de réaction, dans lequel l'adsorbeur est disposé dans la paroi de l'élément tubulaire (1), et/ou
b) un catalyseur servant à transformer de manière catalytique des impuretés dans l'air, dans lequel le catalyseur est disposé dans la paroi de l'élément tubulaire (1).

3. Appareil de purification de l'air ambiant selon l'une quelconque des revendications précédentes,
**caractérisé en ce**
a) **que** l'appareil de purification de l'air présente un support (11) servant à placer l'appareil de purification de l'air ambiant sur un sol,
b) **que** l'élément tubulaire (1) est orienté de manière perpendiculaire par rapport au sol de sorte que l'air ambiant à purifier entre de manière perpendiculaire dans l'élément tubulaire (1), et
c) **que** l'air ambiant à purifier entre de manière perpendiculaire depuis le bas dans l'élément tubulaire (1), et
d) **que** la paroi de l'élément tubulaire (1) est cylindrique.

4. Appareil de purification de l'air ambiant selon l'une quelconque des revendications précédentes,
**caractérisé par** un filtre à particules en forme de tuyau flexible, qui entoure à l'extérieur l'élément tubulaire (1) et qui filtre l'air ambiant sortant de la paroi de l'élément tubulaire (1).

5. Appareil de purification de l'air ambiant selon la revendication 4,
**caractérisé en ce**
a) **que** le filtre à particules en forme de tuyau flexible est élastique et est enfilé de manière interchangeable sur l'élément tubulaire (1), et/ou
b) **que** le filtre à particules est un filtre HEPA, et/ou
c) **que** le filtre à particules filtre de l'air ambiant au moins 95 % de toutes les particules avec une taille de particule supérieure à 0,3 µm.

6. Appareil de purification de l'air ambiant selon l'une quelconque des revendications précédentes,
**caractérisé par** un préfiltre (3), à travers lequel l'air ambiant est acheminé avant l'entrée dans le générateur de plasma pour éviter un encrassement du générateur de plasma.

7. Appareil de purification de l'air ambiant selon la revendication 6, **caractérisé en ce que** la soufflante (2) est disposée dans la direction d'écoulement entre le préfiltre (3) et l'élément tubulaire (1) de sorte que la soufflante (2) aspire l'air ambiant à purifier à travers le préfiltre (3) en provenance de l'extérieur et le souffle à l'intérieur de l'élément tubulaire (1).

8. Appareil de purification d'air ambiant selon l'une quelconque des revendications précédentes,
**caractérisé par**
a) une forme de stèle, et/ou
b) une installation d'éclairage servant à produire des effets d'éclairage, et/ou
c) une unité de commande (8), laquelle commande l'installation d'éclairage pour produire des séquences lumineuses multicolores.

9. Appareil de purification de l'air ambiant selon l'une quelconque des revendications précédentes,
**caractérisé par**
a) une première partie de réseau (6) servant à alimenter en courant des composants électriques de l'appareil de purification de l'air ambiant à partir d'un réseau électrique, et/ou
b) une seconde partie de réseau (7) servant à alimenter en courant le générateur de plasma.

10. Appareil de purification de l'air ambiant selon l'une quelconque des revendications précédentes,
**caractérisé par** une gaine (12), qui entoure à l'extérieur l'élément tubulaire (1) et s'étend au moins sur toute la longueur de l'élément tubulaire (1), dans lequel un entrefer entre l'élément tubulaire (1) et la gaine (12) reste dégagé.

11. Appareil de purification de l'air ambiant selon la revendication 10,
**caractérisé en ce**
a) **que** la gaine (12) est cylindrique, ou
b) **que** la gaine (12) est façonnée de manière concave, ou
c) **que** la gaine (12) est façonnée de manière convexe.

12. Appareil de purification d'air ambiant selon l'une quelconque des revendications 10 à 11, **caractérisé en ce que** la gaine (12) ne laisse pas passer l'air, mais présente en bas une ouverture d'entrée d'air et en haut une ouverture de sortie d'air.

13. Appareil de purification d'air selon l'une quelconque des revendications précédentes, **caractérisé par** un porte-modules (4), qui contient la première partie de réseau, la seconde partie de réseau, la soufflante (2), le préfiltre (3) et/ou l'unité de commande.

14. Appareil de purification d'air ambiant selon la revendication 13, **caractérisé en ce que** le porte-modules (4) est disposé au moins en partie dans la gaine (12).
